# EUROPEAN PATENT APPLICATION

(11) **EP 3 178 514 A1**
(43) Date of publication of application: **14.06.2017**
(21) Application number: 15198588.4
(22) Date of filing: 09.12.2015
(51) Int. Cl.: A61M 25/00

(54) **URINARY CATHETER WITH INTEGRATED CONNECTOR**

(71) Applicant: Dentsply IH AB, 431 21 Mölndal (SE)
(72) Inventor: Lovmar, Martin, 431 69 MÖLNDAL (SE)
(74) Representative: Awapatent AB

(57) **Abstract**

A urinary catheter is disclosed, comprising a tubular shaft extending between an insertable end and a flared rearward part with a discharge end, and with an internal lumen extending between at least one drainage opening at or in the vicinity of the insertable end and an outlet opening at the discharge end. The rearward part comprises an outward protrusion formed at the discharge end around the outlet opening. Further, the tubular shaft is an extruded tube and the rearward part is monolithically and integrally connected with the tubular shaft. A method and apparatus for producing such a catheter is also disclosed, involving forming of the flared rearward part at an end of the tubular shaft and forming of the outward protrusion at the end of the flared rearward part simultaneously with the forming of the flared rearward part.

## Description

### Field of the invention

The present invention relates to an apparatus and a method for manufacturing a tubular object, such as a tube or a catheter, for insertion into a body passageway.

### Background of the invention

Many types of medical devices having a tubular object for insertion into a body passageway are known, such as urinary catheters, stents, etc. These tubular objects conventionally comprise an insertable end provided with one or more draining openings such that fluid can enter the tube, and a non-insertable end provided with an outlet being in fluid connection with the draining openings via the tube. The non-insertable end of the tubular object may advantageously be flared, and have the function of a connector for connecting the tubular object to a receptacle.

The present invention in particular relates to a urinary catheter, and in particular for urinary hydrophilic catheters. Urinary catheters are commonly used for draining urine from the bladder. One type of urinary catheters is indwelling catheters, so-called Foley catheters, which are maintained in place in the urethra for an extended period of time, such as for days, weeks or even months. Another type of urinary catheters are intended for short term use, so-called intermittent catheters. Intermittent urinary catheters are used for draining the bladder once, and then be removed. Intermittent catheters are typically used for a few minutes, and catheterization is typically made by the user him/her self, so-called self-catheterization, and is typically performed many times a day. Typically catheters for intermittent catheterization are used by patients suffering from urinary incontinence, suffering from neurogenic bladder dysfunction, by disabled individuals like para- or tetraplegics and the like. Many of the users using self-catheterization have limited or reduced dexterity. Using an intermittent catheter, the bladder may be drained through a natural or artificial urinary canal. Many catheters for intermittent catheterization are provided with a hydrophilic coating or the like, providing a smooth and slippery surface when wetted, for safe and comfortable insertion in the urinary canal.

Various techniques for producing tubular objects with a flared end are known, some of which involves extrusion. For example, GB 744 327 discloses an extrusion process for forming an endless tube. The extruded tube is cut into suitable lengths, and subsequently, a tip portion and a flared end portion are formed at the respective ends. Formation of the flared end is made by pushing the extruded end over a conical or frusto-conical mandrel during simultaneous heating, and the thus formed part is thereafter immediately cooled in order to obtain a permanent deformation. However, this known method is relatively cumbersome and costly, and does not lend itself for fully automated manufacturing. Further, the hereby produced catheter connector is difficult to handle, in particular for users having poor dexterity.

However, although this process allows for manufacturing of catheter tubes with a flared end, there is a need for a more efficient production process, and for improved catheters being produced in this way.

### SUMMARY of the invention

It is therefore an object of the present invention to provide an improved and more efficient process for manufacturing of the tubular objects with a flared end, as well as a corresponding apparatus for manufacturing and an improved urinary catheter.

This object is achieved with a method, apparatus and urinary catheter according to the appended claims.

According to an aspect of the invention there is provided a urinary catheter comprising a tubular shaft extending between an insertable end and a flared rearward part with a discharge end, and with an internal lumen extending between at least one drainage opening at or in the vicinity of the insertable end and an outlet opening at the discharge end, wherein the rearward part comprises an outward protrusion formed at the discharge end around said outlet opening, and wherein the tubular shaft is an extruded tube and the rearward part is monolithically and integrally connected with the tubular shaft.

The term "tubular shaft" as used herein refers to an object at least part of which forms a tube. The term "tube" here refers to an elongated shaft with a lumen therein. The tube may typically be an elongate hollow cylinder, but may also be a hollow shaft of other cross-sectional shapes.

The term "flared" here indicates a shape which expands or opens outwardly, so that the cross-sectional area at least in some parts increases in a direction away from the insertion end. However, this increase in cross-section area need not necessarily occur continuously. On the contrary, the flared rearward part may have an increased cross-sectional area which increases only in one or several sections, and may in other sections remain constant. Further, various sections may have different degrees of increase. Even though the flared rearward end may have a circular cross-section, it is to be acknowledged by the skilled addressee that non-circular cross-sections, such as oval or elliptical cross-sections are also feasible, and may be formed in the same way. Further, the flared rearward part may similarly be formed with varying thickness around its circumference and/or along its length.

The term "outward protrusion" relates to a protruding shape, protruding out from the surface of the catheter, and at least partly protruding in a radial direction. The outward protrusion may be shaped in various ways, such as being in the form of a flange, e.g. having an essentially rectangular shape. However, the outward protrusion may also have a rounded shape. The outward protrusion is preferably formed by solid material, but may also enclose or partly enclose a non-filled cavity. The present invention is based on the realization that by using e.g. a moveable tapered mandrel, a part of an extruded tube can be expanded into a flared end, and also be provided with a protrusion around the discharge end. This facilitates production, and provides a very stable and useable product, which is also more cost-efficient compared to comparable prior art products.

Since the connector is formed as an integrated, monolithic part of the tube, no separate gluing of a connector/rearward part to a shaft is necessary, and there is also a reduced risk for breakage between the shaft and the connector. Still further, since the material used in the shaft and the material used in the connector are preferably the same, and share the same manufacturing history, the properties of these materials are more controllable.

A flared end and the outward protrusion arranged at the end thereof can prevent that the tubular object is pushed so far into the body passageway that the non-insertable end of the tubular object disappears into the body passageway. The flared end with the outward protrusion also makes it easier for an operator to handle the catheter, both for insertion and to get hold of the tubular object when the tubular object should be removed from the body passageway. The flared end with the outward protrusion also facilitates connecting of the rearward part to additional drainage tubes, to a receptacle or the like. This facilitated handling is of particular importance for users having poor dexterity.

In addition, the outward protrusion makes the discharge end as well as the entire rearward part more stable and rigid. This also makes it easier to grip and handle the catheter. It also prevents deformation of the catheter during production, packaging and storage.

The outward protrusions are preferably provided in the form of an outwardly protruding ring, continuously encircling the outlet opening of the discharge end. However, alternatively, one or more protrusion(s) may be arranged along only part of the circumference, such as in one or several arcs or segments around the discharge end.

The transition between the tubular shaft and the preferably flared rearward part may occur smoothly and gradually. However, it is also feasible to provide an additional second protrusion in this transition area. This additional outward protrusion preferably encircles the catheter shaft at or in the vicinity of the transition between the rearward, and preferably flared, rearward end, and the tubular shaft. This additional outward protrusion may have a rounded shape, e.g. in the shape of a semicircle, or a shape having more pronounced edges, e.g. in a rectangular shape. However, many other shapes are also feasible. For example, the protrusion height may be greater towards one end compared to the other end, so that the upper surface of the protrusion being slanted, giving the protrusion a conical or frusto-conical shape.

Such an additional outward protrusion makes it easier to grasp and hold the catheter, thereby improving the grippability and maneuverability of the catheter. It also provides additional measures to prevent too deep insertion of the catheter. It may also be used for connecting additional parts to the catheter, such as arrangement of a tubular insertion aid around the rearward part of the catheter.

The additional outward protrusion may be provided in various ways, and as an integral part of forming the rearward end and the first outward protrusion, or as a separate forming step. For example, the additional outward protrusion may be formed by heating the material to be formed and then forming the material by vacuum forming, by mechanical compression between two clamps or the like, etc.

The urinary catheter is preferably a urinary catheter for intermittent catheterization, i.e. for short time use, such as catheterization of a few minutes duration, being repeated a number of times each day. The term "short term use" indicates a use that is limited in time, and in particular limited to a time period of less than 15 minutes, and preferably less than 10 minutes, and most preferably less than 5 minutes. The catheter of the present invention is excellently suited for self-catheterization, and can safely and easily be used also by inexperienced users, and/or users suffering from poor dexterity. The catheter is further preferably provided with a hydrophilic surface coating provided on at least an insertable part of the catheter. The hydrophilic surface coating exhibits a low friction when wetted with a wetting liquid, such as water or saline.

The urinary catheter may be a male catheter, preferably having a total length within the range of 35-40 cm, and having an insertable length within the range of 20-35 cm. Alternatively, the urinary catheter may be a female catheter, preferably having a total length of 5-20 cm, and having an instertable length within the range of 4-15 cm.

The urinary catheter preferably has only one, single internal lumen extending between the outlet opening and drainage openings arranged close to the proximal, insertable end of the catheter.

One or several discharge openings may be arranged at or in the vicinity of the insertable end. In a preferred embodiment, the discharge openings are arranged in the side wall of the catheter, at some small distance from the insertable end. The insertable end, i.e. the tip of the catheter, is preferably closed, and is preferably formed in a rounded shape. However, alternatively or additionally, a drainage opening may also be formed in the tip.

The insertable end may have an outer diameter which at all places is equal to or lower than the outer diameter of the tubular shaft. Thus, the tip of the catheter may have the same outer diameter as the rest of the tubular shaft, or alternatively have a decreasing diameter towards the tip end, thereby providing e.g. a conical end. However, it is also possible to provide a tip which at least partly has a larger diameter than the diameter of the tubular shaft, thereby providing e.g. a bulbous tip.

The tip may be straight, extending in the same direction as the tubular shaft and forming a rounded forward end, thereby forming a Nelaton type catheter. However, the tip may also be curved, forming a Tiemann or Coudé type catheter. Tiemann and Coudé catheters have a tip which is angled upward, to assist in negotiating the male prostatic curve. Thus, this tip form facilitates passage through the bladder neck in the presence of obstruction e.g. from a slightly enlarged prostate gland (e.g. in benign prostatic hyperplasia), and can be helpful for such and other difficult insertions.

The tip may be formed directly in the forward end of the tubular shaft, e.g. by melting. However, it is also possible to provide a separately manufactured tip, of the same or a different material, and subsequently attaching this tip to the forward end of the tubular shaft. Such a separately produced tip may be manufactured by injection molded, but may also be manufactured in other ways. A separately produced tip may be connected to the tubular shaft by at least one of welding, adhesion and inject molding.

A color marking may further be integrated in the catheter, wherein the color coding represents the dimensional size of the catheter. To this end, a color pigment may be provided in all or part of the material forming the catheter, and different colors may represent different Charrière /French numbers. In a preferred embodiment, the color marking comprises one or several colored strip(s), extending at least partly in the longitudinal direction of the catheter, and preferably extending over the full length of the catheter, from the proximal tip to the discharge end. Such strip(s) may e.g. be provided during extrusion of the tube/tubular shaft by co-extruding a material having therein mixed color pigment of the desired color together with the non-colored or differently colored material forming the rest of the catheter.

The tubular shaft and the flared rearward part are preferably made of the same material, and the material may be a single material, or a mixture or blend of various materials. The material(s) may e.g. be thermoplastic elastomers, such as polyolefin based elastomers, or thermoplastic polymers, such as polyethylene, polypropylene or polyvinyl chloride, or any other material suitable for extrusion.

In order to obtain good properties for handling, painless and easy insertion, etc, the material(s) of the tubular shaft and the tip are preferably prepared and composed in such a way that they fulfill at least some of the following requirements, and preferably essentially all of them:
- The material of the tubular shaft preferably has a hardness adequate for the intended use. Specifically, the micro Shore A hardness should preferably be in the range 75-95, and more preferably in the range 75-90, and most preferably within the range 78-85, for the tubular shaft.
- It is further preferred that the material of the tubular shaft and flared rearward part has a melting temperature exceeding 90 deg. C, and preferably exceeding 110 deg. C, and most preferably exceeding 130 deg. C.
- It is preferred that the material is capable of being sterilized by known sterilization methods. In particular it is preferred that the material has a radiation resistance such that it can endure at least 50 kGy essentially without degradation, in order to enable radiation sterilization of the urinary catheter.
- The material of the tubular shaft should preferably exhibit low resilience.
- The material should preferably have good kinking properties.
- The material is preferably free or essentially free from chlorine or other halogens.
- Preferably, the material comprises essentially only comprise carbon, hydrogen, nitrogen and oxygen. These constituents should in combination preferably exceed 90% in weight of each material, and preferably exceed 95%. The amount of nitrogen is preferably less than 10%, and most preferably less than 5%.
- The material should preferably be biocompatible.

In case different properties are wanted, this may be accomplished with the use of the same material, e.g. by treatment of the materials in different ways, or by modification of the material(s) by using a different blend of polymers, by additives such as plasticizers, medical oil (i.e. oil of a medical grade), paraffin, etc.

The material(s) is preferably a polymer material. The material(s) may be a polymer blend comprising primarily polyolefin, such as at least 80% by weight of polyolefin and therein possibly intermixed medical oil and/or paraffin. Polyolefin is a material comprising olefin monomers, such as one or several of ethylene, propylene, pentene, etc. The polyolefin may comprise at least one polymer selected from the group: polyethylene, polypropylene, and styrene block copolymer (SEBS). The polymer blend may further comprise a composition/polymer having molecules with active hydrogen(s), wherein the composition having molecules with active hydrogen(s) is preferably a polymer or other molecule where the active hydrogen(s) is bound to the polymer via nitrogen. Molecules with active hydrogen(s) are molecules having hydrogen that is prone to react with other substances, and thus to leave its position in the molecule. Examples of such compositions having molecules with active hydrogen groups are alcohols, amides, amines, urethane and acids. The composition having molecules with active hydrogen(s) is preferably a polymer, and preferably at least one of polyamide and polyurethane. However, other polymers having active hydrogen(s) may also be used. Preferably, the polymer blend comprises a weight percentage of the composition having molecules with active hydrogen(s) in the range of 2-20, and preferably in the range 3-15 and most preferably in the range 5-10. Such polymer blends are known from US 2012/0191073 and US 8168249 by the same applicant, said documents hereby being incorporated in their entirety by reference.

However, other materials may also be used, such as polyurethanes, latex rubbers, silicon rubbers, other rubbers, polyvinylchloride (PVC), other vinyl polymers, polyesters, polyacrylates, polyamides, polyolefines, thermoplastic elastomers, styrene block copolymers (SEBS), or polyether block amide (PEBA), and combinations of these. Suitable thermoplastic materials are materials such as polyurethane, polyvinyl chloride, polyethylene and other thermo-formable materials.

Still further, the tubular shaft can be made of a degradable material, e.g. of the type disclosed in WO 2011/036162, said document hereby being incorporated in its entirety by reference. The degradable material may e.g. comprise monosaccharide, disaccharide, oligosaccharide and/or polysaccharide. Preferably, the degradable material comprises at least 40 % by weight of monosaccharide, disaccharide, oligosaccharide and/or polysaccharide. It is also preferred that the degradable material primarily comprises water and at least one of sugar and starch, and wherein the degradable material preferably comprises at least 90 % by weight of said constituents. Additives to control consistency and elasticity may also be incorporated, e.g. a collagen based material such as gelatin. Preferably, the degradable material is such that it becomes essentially totally dissolved if maintained in water at room temperature for at least 6 hours.

The catheter is preferably coated with a hydrophilic surface coating, exhibiting a low friction when wetted. The surface coating is preferably provided at least on an insertable part of the catheter. In case both the tip portion and the shaft, or at least a part of the shaft, is provided with the surface coating, the surface coating may be provided on the tip and the shaft prior to connection of these parts, but is preferably provided after this connection. The coating process may be provided in the way discussed in EP 0 799 069 by the same applicant, said document hereby incorporated in its entirety by reference.

The hydrophilic polymer may be at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride, polyactide, polyethylene glycol and copolymers thereof. Preferably, the hydrophilic polymer is polyvinyl pyrrolidone.

The hydrophilic coating preferably forms a polyurea network, and most preferably the polyurea network is arranged to form a covalent bond to active hydrogen groups in the substrate. Alternatively, the hydrophilic coating may form an ester bond or an epoxy bond to active hydrogen groups in the substrate.

According to one embodiment, coating of the substrate material of the catheter may be made by a process comprising the steps of: applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature.

However, other hydrophilic coatings are also feasible, such as a coating comprising hydrophilic polymers cross-linked directly to the substrate. The crosslinking may be effected by means of irradiation, e.g. by electron beams or UV light.

The catheter is preferably arranged in a package, to maintain it sterile prior to use.

The catheter preferably has a radiation resistance such that it can endure at least 50 kGy essentially without degradation. Hereby, radiation sterilization of the medical device can be used, without affecting the properties of the medical device.

According to another aspect of the invention, there is provided a method for producing a medical device, such as a urinary catheter, comprising:
providing an extruded tubular shaft, having an internal lumen therein;
providing an insertable end at one end of said tubular shaft;
forming a flared rearward part at an end of the tubular shaft being opposite to said insertable end;
wherein outward protrusions are formed at the end of the flared rearward part simultaneously with the forming of the flared rearward part.

Hereby, a very advantageous catheter or similar medical device is obtained, as discussed in the foregoing, e.g. being very easy to handle even for persons having poor dexterity. Further, the production method is relatively simple and cost-effective. The tubular shafts can be produced in a conventional way, and be cut into suitable lengths. Thereafter, immediately following the extrusion or at some later time, the flared rearward part and the thereon arranged outward protrusions may be formed.

For example, the tubular shaft can be an extruded standard tube. This is very beneficial, since extruded tubes are very cost-effective to produce, and also have very good properties, such as a very well-defined and even wall thickness.

Further, since the connector is formed as an integrated part of the tube, no separate glueing of connector to a shaft is necessary, and there is also a reduced risk for breakage between the shaft and the connector. Still further, since the material used in the shaft and the material used in the connector, share the same manufacturing history, the properties of these materials are more controllable.

In one embodiment, the flared rearward part is formed by insertion of at least one conical mandrel into the tube end during application of heat. Thus, a relative movement between the tubular shaft and the mandrel pushes the end of the tubular shaft onto the conical mandrel, thereby expanding and flaring the end of the tubular shaft into the shape of the mandrel. The flared rearward part may also be formed by sequential insertion of two or more conical mandrels into the tube end during application of heat, each mandrel having greater dimensions than the previous one. This gradually increased flaring provides less stress on the material.

At least one of said conical mandrel(s) is further preferably provided with a flange at the rear, larger end. This flange forms a stop for the tubular shaft material when being expelled onto the mandrel. Hereby, the outward protrusions are formed at the flange simultaneously with the flaring of the rearward part.

The insertion of the tapered mandrel into the end of the tubular shaft is effected by a relative movement between these parts. This can e.g. be effected by keeping the tubular shaft in a fixed position, e.g. by means of clamping, and then moving the tapered mandrel, preferably in the axial direction of the tubular shaft, into the opening of the tubular shaft. However, it is also possible to keep the tapered mandrel in a fixed position, and instead move the tubular shaft towards the tapered mandrel. For example, the tubular shaft may be held by a clamping arrangement, and the clamping arrangement being moveable in relation to the tapered mandrel. It is also feasible to move both the tapered mandrel and the tubular shaft, either simultaneously or intermittently.

The term "tapered mandrel" here indicates a mandrel with a cross-sectional area that increases in the direction away from the forward end to be inserted into the tubular shaft. For example, the tapered mandrel may have a conical or frusto-conical shape. However, the tapered mandrel may also take other shapes that allow formation of a flared or funnel-shaped end of the tube.

The clamping arrangement for holding the tubular shaft may be arranged to clamp the tubular shaft at some distance from the end to be pushed over the tapered mandrel. However the clamping arrangement may also be provided with an end part, facing the tapered mandrel, and having a shape complementary to the shape of the tapered mandrel, thereby aiding in the flaring of the tubular shaft end. In other words, the clamping arrangement may also have the additional purpose and advantage of assisting in forming the flared end to the desired shape over the mandrel. Thus, the clamping arrangement preferably serves the dual functions of both securing the tubular shaft for insertion onto the mandrel and shaping the flared end into the desired shape.

Heating may e.g. be provided by internal heating of the mandrel, and/or by external application of the heating, e.g. by directing a hot air stream, infrared (IR) radiation or the like towards the mandrel and/or the tubular shaft end. Heating from the outside may be provided by heating elements arranged in contact with the tubular shaft material, or by heating means arranged at a certain distance from the material, i.e. without direct contact. However, many other ways of accomplishing local heating are also feasible.

Further, instead of using a mandrel having a fixed shape, it is also possible to use mandrels that are expandable, which may be inserted into the tubular shaft end, and thereafter, subsequent to heating, may be expanded into a desired shape.

However, other ways of forming the flared rearward part and the outward protrusions thereon are also feasible. For example, it is possible to arrange a tubular shaft in a cavity having walls the inside of which present the desired shape of the flared rearward part, and then increasing the pressure in the tubular shaft during simultaneous heating of the material within the cavity. This method can be exercised in a similar way as blow molding of PET bottles and the like. In a preferred embodiment, the tubular shaft initially have double the length of the catheter intended to be produced. Hereby, the middle of the tubular shaft may be arranged in the cavity, and the cavity may have the internal complementary shape of two flared rearward parts, being arranged back-to-back. Hereby, two catheters are formed at the same time, and the catheters may subsequently be cut apart in the centre, by means of laser cutting or the like. If only one catheter is formed at a time, the end of the inflated part may be cut off and then discarded.

As already mentioned in the foregoing, a color marking may be integrated in the tubular shaft, and this may e.g. be made during extrusion of the tubular shaft, e.g. by co-extruding a colored material together with the non-colored or differently colored material forming the remainder of the tubular shaft.

The method may also comprise forming of tip portion at the end of the tubular shaft being opposite the flared end, and preferably in a forming step involving melting.

The method may also preferably comprise forming of at least one, and preferably two or more, drainage openings in the side wall of the tubular shaft. This may be accomplished by punching, with punching means, or blank cutting.

According to still another aspect of the present invention, there is provided an apparatus for forming a flared rearward part of a tubular medical device, such as a urinary catheter, from an extruded tubular shaft, said apparatus comprising a support arrangement for holding at least one conical mandrel, a clamping arrangement arranged to hold a tubular shaft, and a heating arrangement to supply heat to said tubular shaft when in contact with said mandrel, wherein said support arrangement and said clamping arrangement are moveable in relation to each other, thereby pushing an end of said tubular shaft over said mandrel, and wherein at least one of said mandrel(s) is provided with a rear abutment end to form outward protrusions at the end of the flared rearward part simultaneously with the forming of the flared rearward part.

Hereby, similar advantages and properties are obtained as discussed above in relation to the first and second aspects of the invention.

The above described method and apparatus may be used to produce a tube that constitutes, or is part of, a medical device, such as a catheter, stent, etc. In particular, the above described method and apparatus is suitable in a process for producing urinary catheters.

These and other aspects of the invention will be apparent from and elucidated with reference to the embodiment described hereinafter.

### Brief description of the drawings

These and other aspects of the present invention will now be described in more detail, with reference to the appended drawings showing embodiment(s) of the invention.
Fig. 1a and 1b are perspective views illustrating a catheter according to an embodiment of the invention, where Fig. 1b is an enlarged view of the flared rearward part of the catheter of Fig. 1a;
Fig. 2a and 2b are side views of the catheter of Fig. 1, where Fig. 2b is an enlarged view of the flared rearward part of the catheter of Fig. 2a;
Figs. 3a-h are schematic cross-sectional side views of various embodiments of catheters in accordance with the invention;
Fig. 4 is a schematic cross-sectional side view illustrating a catheter with an integrated color marking, in accordance with an embodiment of the present invention;
Figs. 5a-e are schematic cross-sectional views, seen in a longitudinal direction, illustrating different embodiments of catheters with integrated color markings;
Figs. 6a-d are schematic views illustrating various embodiments of the catheter tip, in accordance with the invention;
Figs. 7a and 7b are top views of the rear end of the catheter, illustrating an outward protrusion being uniform around the outlet opening (Fig. 7a) and being non-uniform around the outlet opening (Fig. 7b);
Fig. 8 is a schematic top view of an apparatus for producing a urinary catheter according to a first embodiment of the invention;
Fig. 9 is a schematic top view of an apparatus for producing a urinary catheter according to a second embodiment of the invention;
Fig. 10 is a schematic view of a die for forming a co-extruded catheter having color markings;
Fig. 11 is a schematic top view of an apparatus for forming a tip in a catheter;
Fig. 12 is a perspective view of a catheter similar to the one in Fig. 4; and
Figs. 13a-d are schematic side views of various embodiments of catheters according to other embodiments, having an additional second outer protrusion.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

The following discussion is in particular concerned with hydrophilic urinary catheters for intermittent use. However, the invention can also be used in relation to other types of urinary catheters. Also, it is to be noted that the same technical teaching may also be used for producing other types of medical devices insertable into a body passageway, such as other types of catheters, stents, etc.

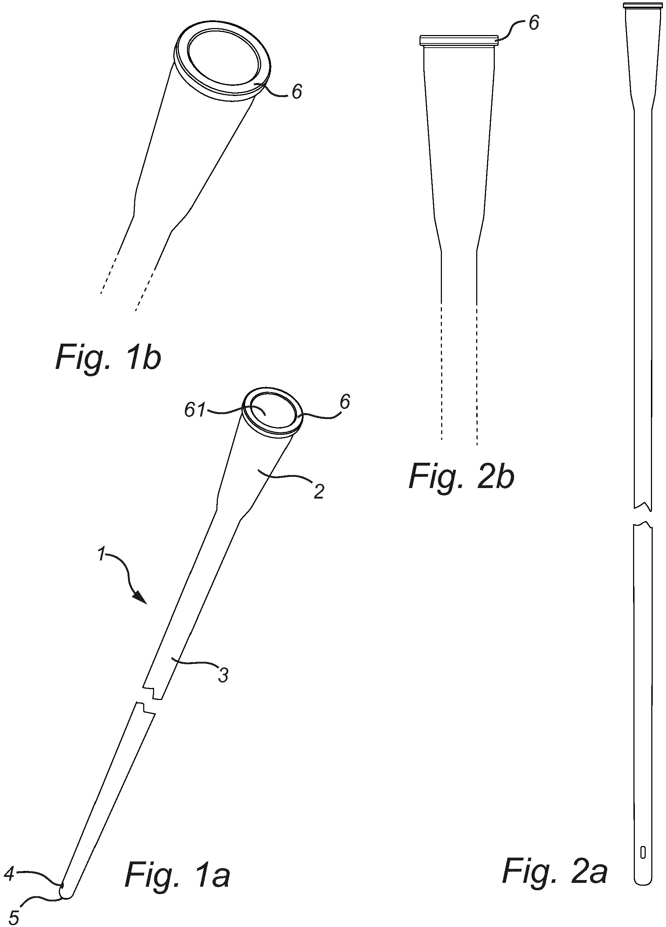

A catheter 1 as illustrated in Figs. 1 and 2, comprises a flared rearward part 2, extending down from the discharge end and the outlet opening 61, and an elongate shaft or tubular shaft 3 projecting forwardly from the rearward part 2. An open-ended internal lumen extends from the rear end of the rearward part 2 to one or more drainage apertures 4 in a forward end of the catheter. At the forward end of the elongate shaft 3, a tip 5 is provided, preferably having a rounded tip end. The rearward part 2 may function as a connector of the catheter 1, being connectable to other devices, such as a urine collection bag, a drainage tube or the like.

Further, an outward protrusion 6 is arranged as a protruding ring encircling the outlet opening of the discharge end of the flared rearward part.

The tubular shaft is made by extrusion, and the flared rearward part 2 is formed as a monolithic, integrated part of the tubular shaft 3.

At least a part of the elongate tube 3 forms an insertable length to be inserted through a body opening of the user, such as the urethra in case of a urinary catheter. By insertable length is normally meant that length of the elongate tube 2 which is insertable into the urethra of the patient during ordinary use. In case a hydrophilic catheter is used, the insertable length is coated with a hydrophilic material, for example PVP, or is made of hydrophilic material. Typically, the insertable length is in the range of 80-140 mm for a female patient and 200-350 mm for a male patient.

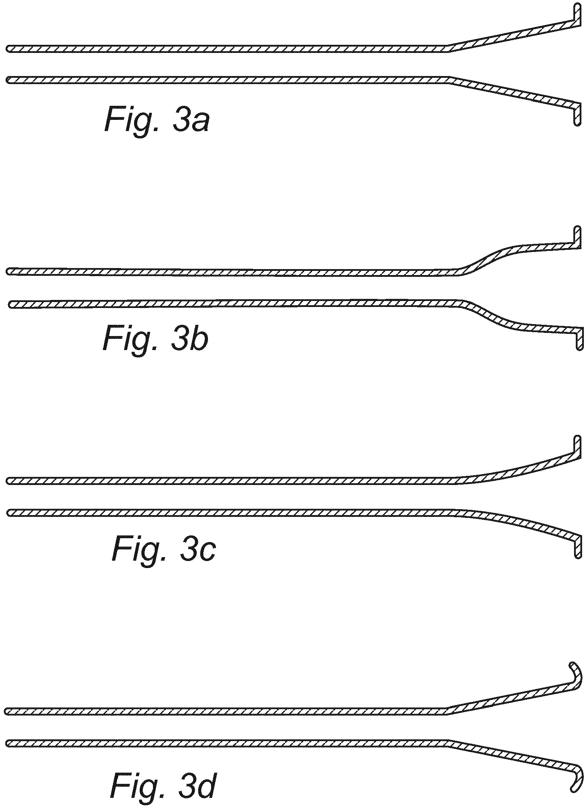

The rearward part and the outward protrusion may be shaped and arranged in various ways. Some examples of this will now be discussed in relation to Figs. 3a-h.

Fig. 3a is an example of a catheter having a continuously flared, conical rearward part. The outward protrusion is here provided as a flange, extending essentially only in a radial direction, and encircling the outlet opening.

Fig. 3b is an example of a catheter having a flared discharge outlet which increases more rapidly in cross-section close to the tubular shaft, and thereafter, towards the outlet opening, less rapidly. The rearward part is hereby in the form of a bowl. The outward protrusion is here again provided as a flange, extending essentially only in a radial direction, and encircling the outlet opening.

Fig. 3c is an example of a catheter having a flared discharge outlet which increases less rapidly in cross-sectional area close to the tubular shaft, and thereafter, towards the outlet opening, more rapidly. The rearward part is hereby in the form of a trumpet. The outward protrusion is here again provided as a flange, extending essentially only in a radial direction, and encircling the outlet opening.

Fig. 3d is an example of a catheter having a conically flared discharge outlet, similar to the one in Fig. 3a, which increases continuously in cross-sectional area. The outward protrusion is here provided as a flange, extending both in a radial direction, and in a direction towards the insertion end of the catheter. Thus, the flange is curved inwardly.

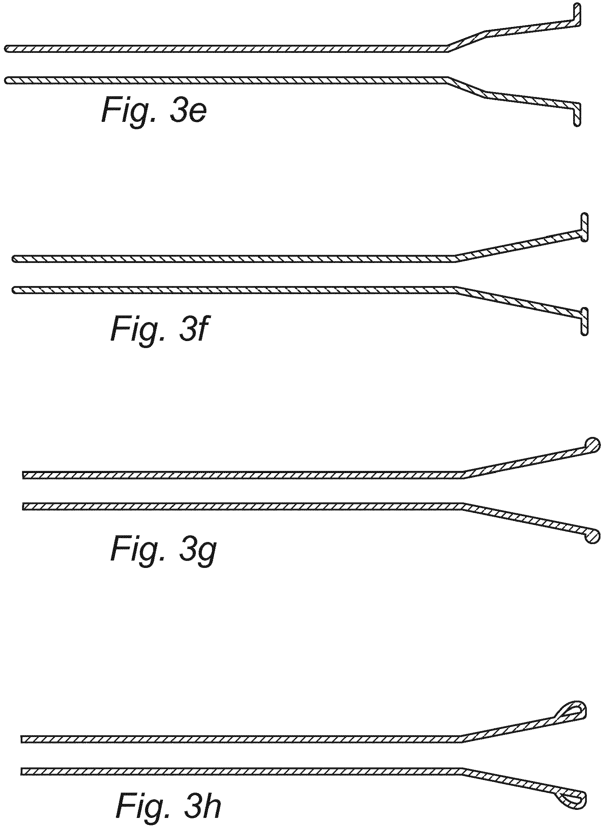

Fig. 3e is an example of a catheter having a flared discharge outlet which increases less rapidly in cross-sectional area close to the tubular shaft, and thereafter, towards the outlet opening, more rapidly. This is similar to the embodiment of Fig. 3b. However, instead of the continuous, smooth transition between the sections, the Fig. 3e embodiment has a distinct first section having a higher increase in cross-sectional area, and a distinct second section, towards the rear end, having less increase in cross-sectional area. The outward protrusion is here again provided as a flange, extending essentially only in a radial direction, and encircling the outlet opening.

In the previous examples, the outward protrusion is extending solely outward from the catheter wall. However, the outward protrusion may, in addition, also protrude inwardly, towards the longitudinal axis of the catheter. A schematic embodiment of this is illustrated in Fig. 3f. The embodiment of Fig. 3f is similar to the embodiment of Fig. 3a. However, here the outward protrusion also protrudes inwardly, thereby providing a flange extending both radially outwards from the catheter wall, as well as protruding, to a slight extent, inwardly. Such an inward protrusion may provide a firmer connection to a urine collection bag, a urine draining tube or the like.

In the previous examples, the outward protrusion is formed as an essentially flat, rectangular flange. However, the outward protrusion may also be formed with rounded corners, or even in the form of a circular or oval protrusion. Such an example is illustrated schematically in Fig. 3g, where the outward protrusion is formed essentially with a circular cross-section.

In the embodiment of Fig. 3g, the outward protrusion is a solid, entirely filed protrusion. However, it is also possible to provide a curved flange, encircling, or partly encircling a hollow cavity. Such an example is illustrated in Fig. 3h, in which the flange is downfolded over the catheter, thereby enclosing a hollow cavity therein. Hereby, a tubular outward protrusion is provided.

However, many other shapes and configurations of the rearward part and the outward protrusions are also feasible, and the discussed embodiments of the rearward parts and the outward protrusions may also be combined in other ways than the ones illustrated here. As a further alternative, it is possible to provide the outward protrusions as two or more protruding rings, arranged at different longitudinal positions, or as protruding knobs or the like, arranged around the discharge end, distributed over the flared rearward part, or arranged in other types of patterns or configurations at the surface of the flanged rearward part.

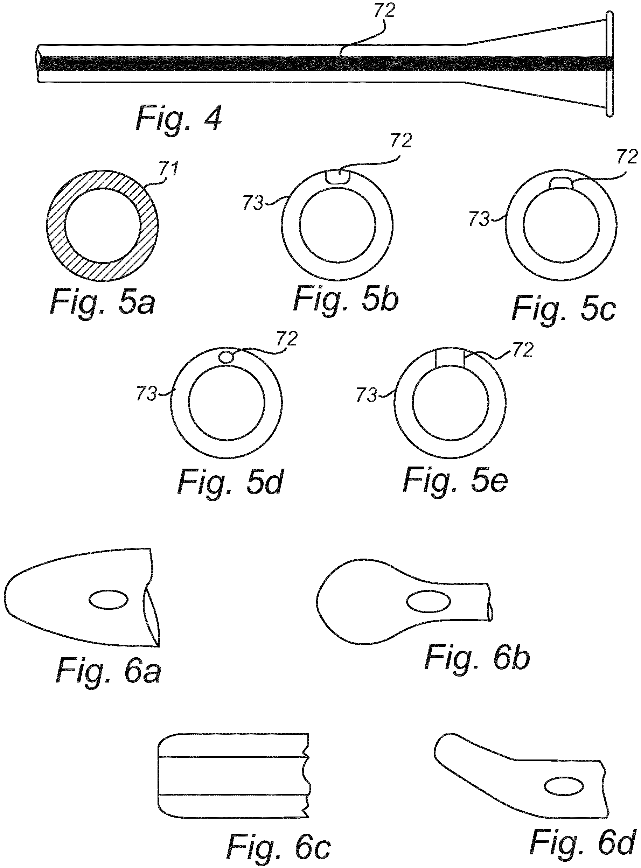

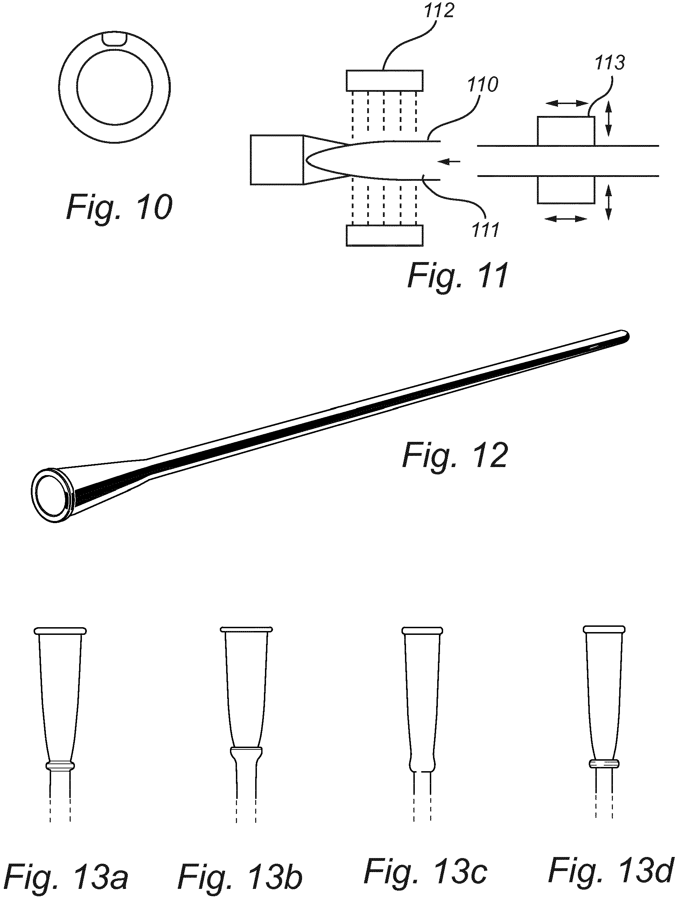

Color markings may be provided on the catheter, indicating e.g. the size of the catheter. Such color markings may be provided by material having color pigment arranged therein. The color markings may e.g. be arranged by co-extrusion, and may e.g. be arranged as a strip 72 in the material, preferably extending over the entire length of the catheter. The strip preferably extends at least partly in the longitudinal direction of the catheter. As one alternative, the strip may extend as a straight line, extending parallel to the longitudinal direction of the catheter. However, the strip may also extend in a serpentine shape, as a zigzag shape or the like. The strip may, as one example, extend in a spiral shape in one or several turns around the catheter. Fig. 4 illustrates, schematically, an embodiment of such a color marking. Here, the strip extends solely in the longitudinal direction of the catheter, and has a uniform thickness. However, it is also feasible that the strip has an increasing width at the flared rearward end. Such an embodiment is illustrated in Fig. 12.

Fig. 5b illustrates how such a color marking may be integrated as a co-extruded strip 72 in a non-colored or differently colored material 73 used in the rest of the tubular shaft. The colored strip 72 is preferably made integral and embedded in the tube material 73, so that the outside of the tube is completely smooth. The colored material is preferably the same material as the non-colored or differently colored material, but with a uniformely dispersed pigment therein, such as a finely powdered pigment. The pigment should preferably be water insoluble and non-toxic. The strip 72 in Fig. 5b is embedded in the catheter material 73, but with one side facing and being in contact with or forming part of the exterior surface of the catheter. Alternatively, as shown in Fig. 5c, the strip 72 may be embedded in the catheter material 73, but with one side facing and being in contact with or forming part of the interior surface of the lumen of the catheter. However, it is also possible to have the strip 72 embedded in the catheter material 73 without contact with any of the catheter surfaces, as shown in Fig. 5d, or with contact or forming part of both the exterior and interior surfaces of the catheter, as shown in Fig. 5e.

Even though the previously discussed examples comprises a single colored strip, more than one strip may also be provided, and then preferably separately distributed around the circumference of the catheter.

However, it is also possible to provide color marking 71 around the full circumference of the catheter shaft, and/or rearward part. Such an embodiment is illustrated in Fig. 5a. In this embodiment, the whole catheter may preferably be colored in the desired color to provide the color marking.

The tip 5 of the catheter may be provided in various ways. The tip may be formed directly on the tubular shaft, e.g. by melting, thereby providing a tip which is monolithically and integrally connected to the tubular shaft. However, alternatively the tip may be a separately produced tip, e.g. formed by injection molding, which is connected to the end of the tubular shaft by gluing, welding or the like.

Some various embodiments of the tip part will now be discussed with reference to Figs. 6a-d.

In one embodiment, illustrated in Fig. 6a, the tip is straight, extending in the same direction as the tubular shaft and forming a rounded forward end. This tip type is generally known as a Nelaton type catheter. In such an embodiment, the tip preferably has an outer diameter which at all places is equal to or lower than the outer diameter of the tubular shaft. The tip is preferably arranged conically tapering in the forward direction, to end in a rounded tip.

In another embodiment, as illustrated in Fig. 6b, the tip is enlarged, thereby presenting at least a part having a larger outer diameter than the catheter shaft. For example, enlarged tip may be in the form of a bulb or the like.

In the embodiments of Fig. 6a and 6b, the catheter tip is provided with a rounded tip, closing of the forward end of the catheter. Drainage openings are provided as openings, so-called catheter eyes, in the sidewall of the tip and/or in the forward end of the elongate shaft. However, it is also possible to use, additionally or as an alternative, a drainage opening being centrally arranged along the longitudinal axis of the catheter. Such an embodiment is illustrated in Fig. 6c. Here, the forward end of the catheter and tip are not closed, and the drainage opening is formed in the extension of the internal lumen of the catheter.

Also, the tip may be curved, forming a Tiemann or Coudé type catheter. Such an embodiment is illustrated in Fig. 6d.

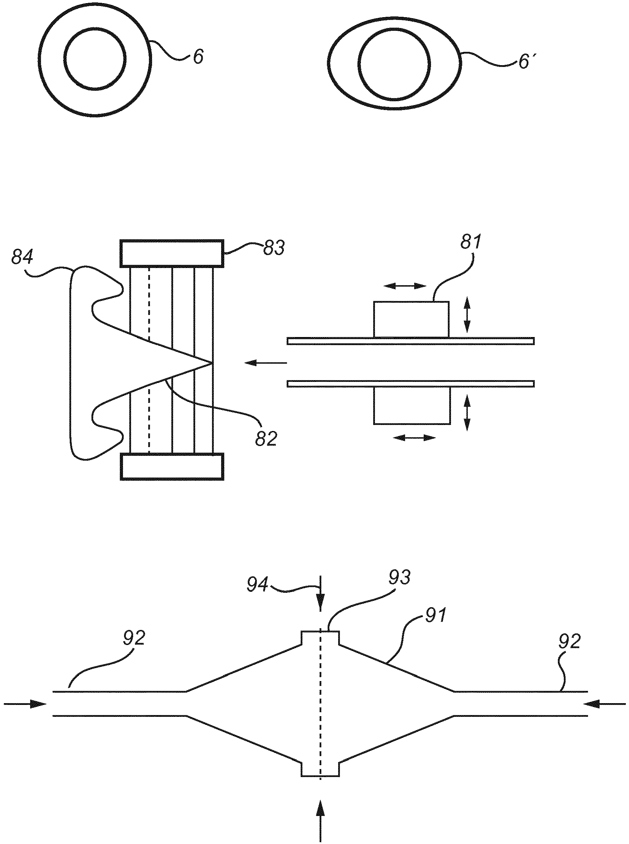

The outward protrusion 6 may, as discussed and illustrated in the foregoing, be arranged as a flange continuously encircling the rearward end of the catheter. The flange may extend solely radially, or both radially and in an axial direction. Further, the flange may be flat, rectangular, or be curved in e.g. a circular or oval shape. It may also be provided as a solid flange, or as a hollow flange. Such embodiments have been discussed in the foregoing. These exemplary embodiments all had an outward protrusion having a uniform width in the radial direction, thereby providing a catheter outlet having a uniform and symmetric shape. Such a uniform shape around the circumference is illustrated in Fig. 7a. However, non-uniform shapes around the circumference are also feasible. For example, the outward protrusion may be provided at only some sections around the circumference, and preferably uniformly distributed around the circumference. One such example is illustrated in Fig. 7b. Here, the outward protrusion 6' comprises two, oppositely arranged outward protrusion sections, and the width of these sections gradually decreases towards the intermediate area there between, in which there is no outward protrusions, or alternatively outward protrusions having a very limited width. Thus, instead of being arranged as a circular flange, being defined by circular inner and outer circumferences and a uniform width there between, the outward protrusion is here formed as an oval or elliptic flange, having an elliptic or oval outer circumference and a circular inner circumference, and a non-uniform width there between. Here, the width decreases and increases gradually, so that two oppositely arranged first points around the circumference have maximum width, and two oppositely arranged intermediate second points between said first points have minimum width.

Even though the flared rearward end may have a circular cross-section, non-circular cross-sections, such as oval or elliptical cross-sections are also feasible, and may be formed in the same way. Thus, the outward protrusion may in such an example also have e.g. an oval or elliptic shape, and still have a uniform width.

The catheters may be made in various lengths and dimensions. Typically, the length of the catheter shafts for female catheters are in the range 50-200mm, such as with a length in the size of about 8, 10 or 12 cm, preferably corresponding to a total catheter length of about 11, 13 or 15 cm, and for male catheters may preferably in a length in the range of 180-450 mm, such as in the size of about 32, 35 or 37 cm, preferably corresponding to a total catheter length of about 36, 38 or 40 cm, respectively. The indicated shaft lengths here refer to the length of the catheter tube excluding the flared end, whereas the total catheter length is the length of the shaft and the flared rearward end in combination. The outer diameter of the tube after drying/cooling is preferably in the range 2-15 mm, and more preferably in the range 3-10 mm, and most preferably in the range 4-6 mm, such as 5 mm. The inner diameter of the tube after drying/cooling is preferably in the range 1-10 mm, and more preferably in the range 1.5-6 mm, and most preferably in the range 2-4 mm, such as 3 mm. The thickness of the tube after drying/cooling is preferably in the range 0.4-4 mm, and more preferably in the range 0.5-2 mm, and most preferably in the range 0.6-1.0 mm. The flared rearward end preferably has an inner diameter at its largest end in the range 5-15 mm, and more preferably in the range 6-12 mm, and most preferably in the range 8-11 mm, such as 8.7 mm. The length of the flared rearward part is preferably in the range 10-100 mm, and more preferably in the range 20-60 mm, and most preferably in the range 25-50 mm, such as 35 mm.

The catheter can be produced in various ways, and some exemplary embodiments of methods and production apparatuses to this end will be discussed in more detail in the following.

A tubular shaft is provided by extrusion, using a conventional extruder (not shown). Extrusion of tubes is per se known, and need not be further discussed. The tubular shafts may also be provided by cutting pre-produced and commercially available standard tubes.

In case a co-extruded color marking is to be used, the extrusion die may have two outlet openings, i.e. forming a bi-orifice tubular extrusion die, and arranged in a cross-sectional shape corresponding to the cross-sectional shape of the tube to be produced. An example of such a die is illustrated in Fig. 10.

The insertable end may be provided in the tubular shaft in various ways. One end of the tubular shaft may be formed into a suitable insertion tip, by grinding, melting or the like. Alternatively, a preformed tip, made e.g. injection molding, may be fixedly attached to the end of the tubular shaft.

In one embodiment, as illustrated in Fig. 11, the catheter tip is formed by melt forming. Here, a forming tool or mold 110 is provided with a mold cavity 111, having the desired shape of the tip to be formed. A heating device 112 is further provided for heating of the tubular shaft material, at least in the vicinity of the end to be formed. The heating device may operate in various ways. For example, the mold in itself may be heated, e.g. by running a current through the material of the mold. Additionally or alternatively, heating by means of induction, IR radiation, a hot air stream or the like may be used. It is also possible to pre-heat the end of the tubular shaft.

The tubular shaft is held by a clamping arrangement 113, arranged to hold the tubular shaft, and which enables a relative movement in relation to the mold cavity. After or during heating of the end of the tubular shaft, the tubular shaft is then moved into the mold cavity, thereby melting and deforming the end to its desired shape.

In case more complicated tip shapes are desired, it is also possible to use two or more shaping stations, each having a mold cavity shaped to provide a desired additional deformation of the tip to be formed.

When a pre-formed tip is attached to the tubular shaft, drainage openings may be formed in the tip during formation of the tip. Further, if a central, forward opening is desired, the shaping of the tip is preferably made in such a way that a central opening is maintained. However, if e.g. melting and deformation is used to form a tip with a closed, rounded tip, additional drainage openings in the sidewall of the tip and/or the catheter shaft are often required. Such additional openings may be formed by means of punching, drilling, melting or the like, in a per se known way.

The flared rearward part may also be formed in various ways. In a first embodiment, the tubular shaft is held by a clamping arrangement 81. A conical mandrel 82 is further provided, having a conical end to form the flaring of the end of the tubular shaft. The clamping arrangement and the mandrel are movable in relation to each other, so that the clamping arrangement is moveable back and forth towards the mandrel, which may be fixed, or so that the mandrel is movable back and forth towards the clamping arrangement, which may be fixed. Alternatively, both the mandrel and the clamping arrangement may be moveable.

A heating arrangement 83 is further provided, for heating of the tubular shaft end during, or prior to being pushed over the mandrel. The heating arrangement may operate in various ways. For example, the mold in itself may be heated, e.g. by running a current through the material of the mold. Additionally or alternatively, heating by means of induction, IR radiation, a hot air stream or the like may be used. It is also possible to pre-heat the end of the tubular shaft. In a preferred embodiment, a tubular heating sleeve is provided over the mandrel, providing a heated zone over the mandrel.

Movement of the tubular shaft end over the mandrel causes expansion and flaring of the end of the tubular shaft into the shape of the mandrel.

The mandrel is further provided with a flange 84 at the rear, larger end. This flange forms a stop for the tubular shaft material when being expelled onto the mandrel. Hereby, the outward protrusions are formed at the flange simultaneously with the flaring of the discharge end.

The flared discharge end may also be formed by sequential, successive insertion of two or more conical mandrels into the tube end during application of heat, each mandrel having greater dimensions than the previous one. In such an embodiment, the flange 84 may be provided only on one of the mandrels, or on several or all of them.

Further, instead of using a mandrel having a fixed shape, it is also possible to use mandrels that are expandable, which may be inserted into the tubular shaft end, and thereafter, subsequent to heating, may be expanded into a desired shape.

However, other ways of forming the flared discharge end and the outward protrusions thereon are also feasible. One such alternative embodiment will now be discussed with reference to Fig. 9.

In this embodiment, a mold cavity 91 is provided having interior walls which present the desired shape of the flared discharge end. Preferably, the mold cavity has the internal complementary shape of two flared discharge ends, being arranged back-to-back. The mold is provided with at least one, and preferably two, inlets 92 for receiving a tubular shaft. A tubular shaft is arranged within the mold, so that the tubular shaft extends out from the inlet(s), and the part to be shaped is arranged within the cavity. The material in the cavity is heated, preferably by heating the whole mold. The pressure within the tubular shaft is then increased, by providing an overpressure at one or both external ends of the tubular shaft. Hereby, the part of the tubular shaft being inside the mold cavity is expanded into the interior shape of the mold cavity. This process is similar to blow molding techniques, used e.g. when forming PET bottles and the like. The mold cavity is further provided with an internal shape 93 corresponding to the outward protrusions to be formed in the flared part of the tubular shaft.

After formation, the two parts are separated, e.g. by means of laser cutting device 94 or the like.

The above described method and apparatus may be used to produce a tube that constitutes, or is part of, a medical device, such as a catheter, stent, etc. In particular, the above described method and apparatus is suitable in a process for producing urinary catheters. However, the method and apparatus may also be used for other types of medical devices having a similar shape.

The heating of the tubular shaft for forming the tip and/or the flared rearward end is dependent on the material used in the tubular shaft. However, preferably the temperature is below the melting temperature of the material. Preferably, the material is heated to a temperature within the range 100-130 deg. C, and preferably within the range 105-120 deg. C.

In the embodiments disclosed so far, the transition between the tubular shaft and the preferably flared rearward part occurs smoothly and gradually. However, it is also feasible to provide an additional second protrusion in this transition area, as shown schematically in Figs. 13 a-d. This additional outward protrusion preferably encircles the catheter shaft at or in the vicinity of the transition between the rearward, and preferably flared, rearward end, and the tubular shaft.

This additional outward protrusion may have different shapes. In one alternative, shown in Fig. 13a, the protrusion is formed as a ring having a rounded shape, essentially in the shape of a semicircle. A similar shape is shown in the view of Fig. 13c. However, the protrusion ring may also have more pronounced edges, e.g. in a rectangular shape or in a rectangular shape with beveled or rounded edges. Such an embodiment is illustrated in Fig. 13d.

In the above-discussed embodiments the protrusion ring is symmetrical, with a height distribution being equal on both sides seen from the center of the protrusion ring when viewed in lateral cross-section, i.e. the upper, outer surface of the protrusion ring is essentially flat, and parallel to the longitudinal direction of the catheter, or rounded or slanted, with symmetrically decreasing height towards the tip of the catheter and the discharge end of the catheter, respectively.

However, the protrusion ring may also have a non-symmetric height distribution. For example, the upper, outer surface may be slanted or beveled, and the protruding ring may have a greater height towards the discharge end and a lesser height towards the tip, or the other way around. Thus, in this embodiment the protrusion has a conical or frusto-conical shape. Such an embodiment is illustrated in Fig. 13b.

The additional outward protrusion may be provided in various ways, and as an integral part of forming the rearward end and the first outward protrusion, or as a separate forming step. For example, the additional outward protrusion may be formed by heating the material to be formed and then forming the material by vacuum forming, by mechanical compression between two clamps or the like, etc.

The person skilled in the art realizes that the present invention by no means is limited to the preferred embodiments described above. On the contrary, many modifications and variations are possible within the scope of the appended claims. For instance, the flared rearward part need not be continuously tapering, but may have other geometrical shapes. Further, the tip may be either straight, pointing directly in the longitudinal direction of the catheter, or be slightly curved, so that the end of the tip points in a direction which is non-parallel to the longitudinal direction of the catheter. Further, many different materials and material combinations may be used to produce the tubular shaft and the rearward part, and still obtain the desired material properties. Still further, the access openings/drainage eyes may be provided in either the tip or in the forward end of the tubular shaft, or even in both. Such and other modifications should be construed to fall within the scope of the appended claims.

## Claims

1. A urinary catheter comprising a tubular shaft extending between an insertable end and a flared rearward part with a discharge end, and with an internal lumen extending between at least one drainage opening at or in the vicinity of the insertable end and an outlet opening at the discharge end, wherein the rearward part comprises an outward protrusion formed at the discharge end around said outlet opening, and wherein the tubular shaft is an extruded tube and the rearward part is monolithically and integrally connected with the tubular shaft.

2. The urinary catheter of claim 1, wherein the outward protrusion is provided in the form of an outwardly protruding ring, continuously encircling the outlet opening of the discharge end.

3. The urinary catheter of claim 1 or 2, further comprising an additional second outward protrusion at or in the vicinity of the transition between the rearward part and the tubular shaft, said additional second outward protrusion preferably being provided in the form of an outwardly protruding ring, continuously encircling the catheter.

4. The urinary catheter of any one of the preceding claims, wherein the insertable end has an outer diameter which at all places is equal to or lower than the outer diameter of the tubular shaft.

5. The urinary catheter of any one of the preceding claims, wherein the catheter is at least partly coated with a hydrophilic surface coating, the coating preferably covering at least an insertable part of the catheter, said hydrophilic surface coating exhibiting a low friction when wetted.

6. The urinary catheter of any one of the preceding claims, wherein a color marking is further integrated in the catheter, said color coding representing the dimensional size of the catheter.

7. The urinary catheter of claim 6, wherein the color marking comprises at least one colored strip, extending at least partly in the longitudinal direction of the catheter, and preferably extending over the full length of the catheter.

8. A method for producing a medical device, such as a urinary catheter, comprising:
providing an extruded tubular shaft, having an internal lumen therein;
providing an insertable end at one end of said tubular shaft;
forming a flared rearward part at an end of the tubular shaft being opposite to said insertable end;
wherein an outward protrusion is formed at the end of the flared rearward part simultaneously with the forming of the flared rearward part.

9. The method of claim 8, wherein the flared rearward part is formed by insertion of at least one conical mandrel into the tube end during application of heat.

10. The method of claim 9, wherein the flared rearward part is formed by sequential insertion of two or more conical mandrels into the tube end during application of heat, each mandrel having greater dimensions than the previous one.

11. The method of claim 9 or 10, wherein at least one of said at least one conical mandrel(s) is further provided with a rear abutment end, such as a flange, at the rear, larger end, the flange forming a stop for the tubular shaft material when being expelled onto the mandrel, thereby to form the outward protrusions simultaneously with forming the flaring of the rearward part.

12. The method of claim 8, wherein the flared rearward part is formed by insertion of at least a part of the tubular shaft into a cavity having walls the inside of which present the desired shape of the flared rearward part, and then increasing the pressure in the tubular shaft during simultaneous heating of the material within the cavity.

13. The method of claim 12, wherein the tubular shaft initially has two times the length of the catheter intended to be produced, and wherein the middle of the tubular shaft is arranged in the cavity, and the cavity has the internal complementary shape of two flared rearward parts, being arranged back-to-back.

14. The method of any one of the claims 8-10, wherein a color marking is further integrated in the catheter by co-extruding a colored material together with a non-colored or differently colored material forming the reminder of said tubular shaft, said color coding representing the dimensional size of the catheter.

15. An apparatus for forming a flared rearward part of a tubular medical device, such as a urinary catheter, from an extruded tubular shaft, said apparatus comprising a support arrangement for holding at least one conical mandrel, a clamping arrangement arranged to hold a tubular shaft, and a heating arrangement to supply heat to said tubular shaft when in contact with said mandrel, wherein said support arrangement and said clamping arrangement are moveable in relation to each other, thereby pushing an end of said tubular shaft over said mandrel, and wherein at least one of said mandrel(s) is provided with a rear abutment end to form an outward protrusion at the end of the flared rearward part simultaneously with the forming of the flared rearward part.
